# EUROPEAN PATENT APPLICATION

(11) **EP 2 145 648 A1**
(43) Date of publication of application: **20.01.2010**
(21) Application number: 09165717.1
(22) Date of filing: 16.07.2009
(51) Int. Cl.: A61N 1/05

(54) **Blended coiled cable**

(30) Priority: 16.07.2008 US 81146 P
(71) Applicant: Greatbatch Ltd., Clarence, NY 14031 (US)
(72) Inventor: Kaplan, Paula, St Paul, MN 55116 (US); Kidder, Allison, Boston, MA 02163 (US); Zweber, Jeffrey, St. Louis Park, MN 55426 (US); Brainard, Scott, Columbia Heights, MN 55421 (US); Elhard, John, Chanhassen, MN 55317 (US)
(74) Representative: Duckett, Anthony Joseph

(57) **Abstract**

A cabled lead comprising a first conductor cable having outer strands and a core strand is described. Each of the outer strands and core strand has outer wires that surround a core wire. The outer wires are made of a first material. The core wires have a tube, which may be in the form of a layer, and a core, and the core is disposed in the tube. The tube is made of the first material and the core is made of a second material. The first conductor cable is positioned in a first conductor sheath. The cabled lead has a second conductor cable which may be substantially structurally identical to the first conductor cable and is positioned in a second conductor sheath. The first and second conductor cables are helically coiled to form the cabled lead. In other embodiments, there may be one or more than two conductor cables.

## Description

### CROSS-REFERENCE TO RELATED APPLICATION

This application claims priority to U.S. Provisional Application Serial No. 60/081,146, filed July 16, 2008.

### BACKGROUND OF THE INVENTION

The present invention relates to leads, and in particular medical leads. Medical leads are implanted in a target area of a tissue for stimulation or sensing purposes. For example, the medical lead may be used to deliver electrical stimulation to the surrounding body tissue in which it is embedded, or may be used to detect or otherwise sense the electrical signals produced by the surrounding body tissue in which it is embedded. Medical leads are frequently used in cardiac applications, neurological applications, peripheral nerve stimulation applications, and spinal applications.

Medicals leads need to be flexible and strong in order to withstand the constant bending and flexing of the body in which it is implanted. As a result, medical leads currently in use have large diameters. However, a lead with a large diameter is undesirable, because an invasive surgical procedure is required to initially implant the medical lead in the body of the patient. This exposes the patient to the well know risks associated with invasive surgery, for example infections and healing problems. In addition, if the medical lead breaks or malfunctions after being implanted, the patient will be subjected to another invasive surgical procedure to remove the old lead and implant a new lead. This may be especially problematic in instances when an elongated lead has been implanted, for example a lead implanted in a spine. These elongated leads are prone to failing, because elongated leads have insufficient strength and flexibility for use in such applications.

Thus, there is a need for a medical lead that is flexible and strong, resistant to fatigue, and which has a small diameter. There is also a need for an implantable lead that minimizes the invasiveness of the surgical procedure required to implant or remove the medical lead from the body tissue in which it has been implanted.

### SUMMARY OF THE INVENTION

The present blended coiled cable provides for a cabled lead having a small diameter, high flexibility, high conductivity, high strength and resistance to fatigue, and may be made to have any desired length. In one of the preferred embodiments the cabled lead has a first conductor cable that is housed in a first conductor sheath made of an insulating material. In one of the preferred embodiments, the first conductor cable has six outer strands that surround a core strand, and the six outer strands may be twisted around the core strand in a rope-like fashion. Each of the outer strands and core strand has six outer wires that surround a core wire. With respect to each outer stand and core strand, the outer wires may be twisted around the core wire in a rope-like fashion. The outer wires in each of the strands and core strand are made of a first material. The core wires in each of the outer strands and the core strand include a core made of a second material surrounded by a tube or layer made of the first material. Thus, the first conductor cable is blended in that it comprises two different materials. A second conductor cable housed in a second conductor sheath is provided. The second conductor cable is substantially structurally identical to the first conductor cable and the second conductor sheath is substantially structurally identical to the first conductor sheath. The first and second conductor cables are, in one of the preferred embodiments, helically wound to form a bifilar lead.

In another preferred embodiment, a third conductor cable or a fourth conductor cable may be provided, and each is housed in a sheath and each is substantially identical to the first and second conductor cables. The cables may be helically wound to form trifilar and quadfilar leads.

In another preferred embodiment, there is a strand which includes seven core wires and twelve outer wires. The core and outer wires are arranged relative to one another such that the outer wires contact outer wires and core wires, and the core wires only contact outer wires.

### BRIEF DESCRIPTION OF THE DRAWINGS

Fig. 1 is an isometric view of the cabled lead.
Fig. 2 is a front elevational view of the cabled lead.
Fig. 3 is an enlarged view of detail A of Fig. 2.
Fig. 4 is an enlarged view of detail B of Fig. 3.
Fig. 5 is enlarged front elevational view of an end portion of the cabled lead.
Fig. 6 is an enlarged end view of an embodiment of a multi-cored strand.

### DETAILED DESCRIPTION OF THE PREFERRED EMBODIMENTS

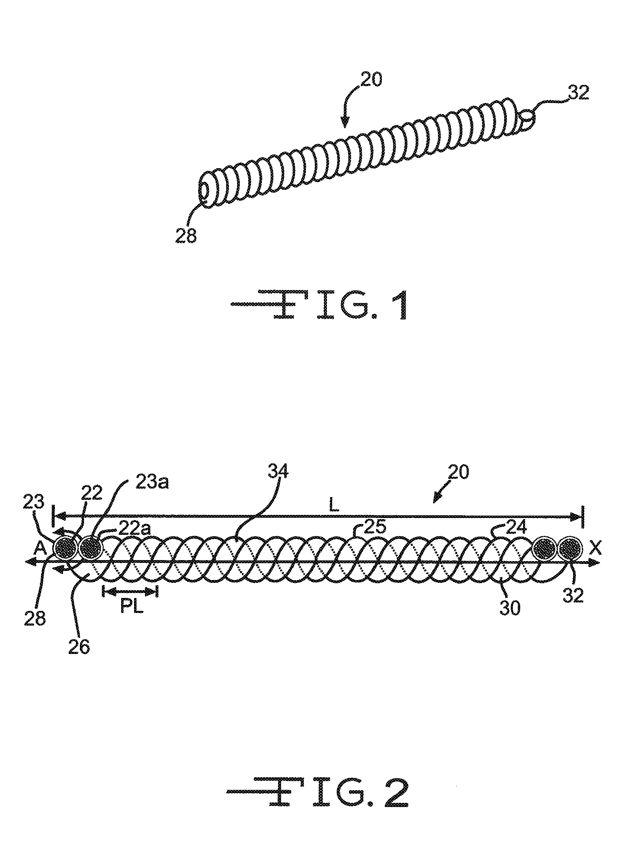

Figs. 1 and 2 show a cabled lead 20 having a first conductor cable 22 housed in a first conductor sheath 23, and a second conductor cable 22a housed in a second conductor sheath 23a. The cabled lead 20 has a distal end portion 26 with a distal end 28, a proximal end portion 30 with a proximal end 32, and an intermediate portion 34 that extends from the distal end portion 26 to the proximal end portion 30. The first and second conductor sheaths 23 and 23a are, in one of the preferred embodiments, made of an insulating material, for example rubber, plastic, or ethylene tetrafluoroethylene copolymer (hereinafter ETFE).

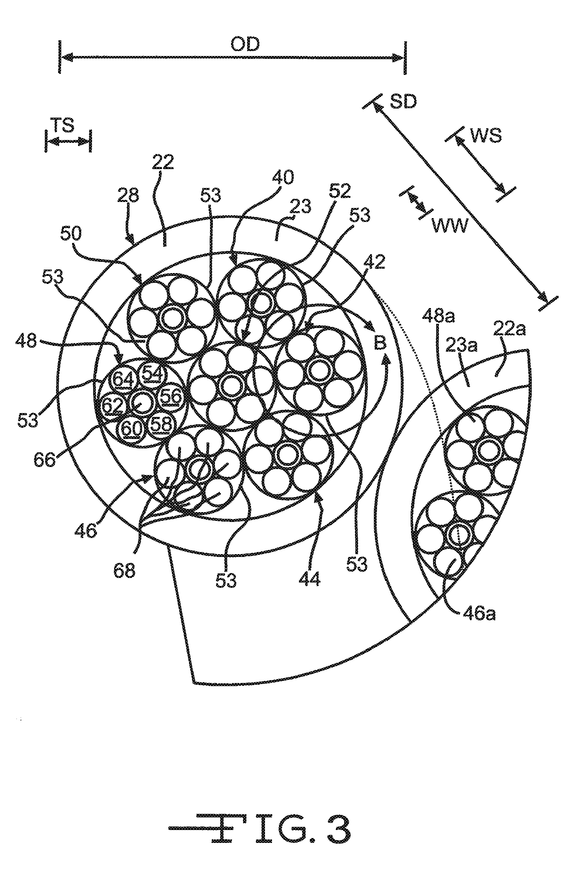

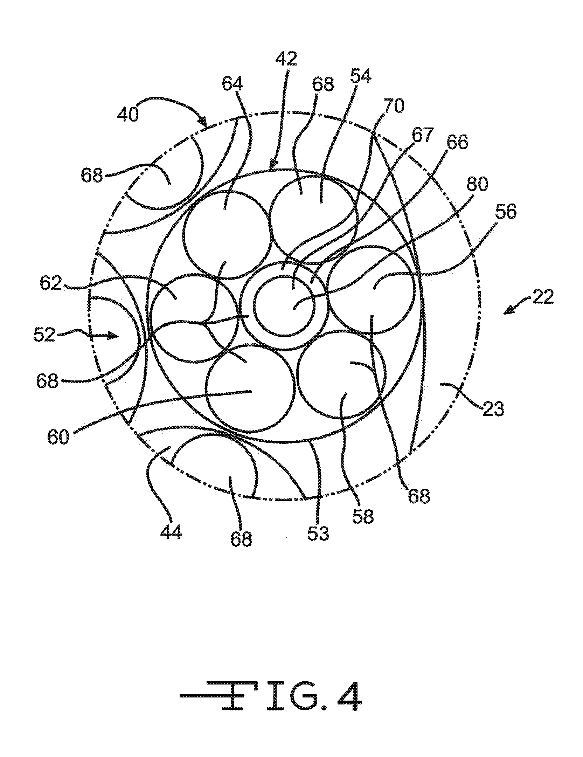

Turning now to Figs. 3 and 4, in one of the preferred embodiments the first conductor cable 22 has six outer strands 40, 42, 44, 46, 48 and 50 and a core strand 52, all of which are substantially identical. The core strand 52 is centrally positioned in the first conductor cable 22, such that it is surrounded by the six outer strands 40, 42, 44, 46, 48 and 50. In one of the preferred embodiments, the outer strands 40, 42, 44, 46, 48 and 50 may be twisted around the core strand 52 in a rope-like fashion. Each of the outer strands 40, 42, 44, 46, 48 and 50, and core strand 52 may be surrounded by a strand insulation sheath 53. The strand insulation sheath 53 may be in the form of a coating or sheath. In one of the preferred embodiments, the strand insulation sheath 53 is ETFE, and may be made of the same materials from which the first conductor sheath 23 is made. The first conductor sheath 23 contacts the strand insulation sheaths 53 surrounding the six outer strands 40, 42, 44, 46, 48 and 50. Alternatively, in an embodiment wherein there are no strand insulation sheaths 53, the first conductor sheath 23 makes direct contact with the outer strands 40, 42, 44, 46, 48 and 50.

Each of the outer strands 40, 42, 44, 46, 48 and 50, and the core strand 52 has six outer wires 54, 56, 58, 60, 62 and 64 that surround a core wire 66. In one of the preferred embodiments the outer wires 54, 56, 58, 60, 62 and 64 may be twisted around the core wire 66 in a rope-like fashion. In Fig. 3, the outer strand 48, the outer wires 54, 56, 58, 60, 62 and 64, and core wire 66 are identified. It is to be understood that each of the outer strands 40, 42, 44, 46 and 50, and the core strand 52 has a substantially identical wire configuration as outer strand 48, but the wires in the outer strands 40, 42, 44, 46 and 50, and the core strand 52 have not been numbered for the sake of clarity. Thus, each of the outer strands 40, 42, 44, 46, 48 and 50 and the core strand 52 has seven wires (six outer wires 54, 56, 58, 60, 62 and 64 and one core wire 66). The configuration of the first conductor cable 22 may generally be described as being a 7 x 7 configuration, because there are six outer strands and the core strand (40, 42, 44, 46, 48, 50 and 52), each, in turn, having six outer wires and a core wire (54, 56, 58, 60, 62, 64 and 66), for a combined total of 49 outer and core wires.

Each of the outer wires 54, 56, 58, 60, 62 and 64, and the core wire 66 has a diameter of 0.0008 inches, designated WW in Fig. 3. The diameter WW may be about 0.0008 inches in other preferred embodiments. The diameter, designated WS, of each of the outer strands and the core strand 40, 42, 44, 46, 48, 50 and 52 is about 0.0024 inches. The diameter WS may be about 0.0024 in other preferred embodiments. The overall diameter of the first conductor cable 22 designated SD is about 0.0072 inches. In another preferred embodiment, the diameter SD of the first conductor cable 22 may be in the range from about 0.008 inches to about 0.0064 inches. Thus, the overall diameter of the first conductor cable 22 is very small, and therefore it may be implanted during a surgical procedure with a minimal level of invasiveness.

The first conductor sheath 23 has a thickness, designated TS in Fig. 3, of about 0.001 inches. The thickness of the first conductor sheath 23 may more or less than about 0.001 inches in other preferred embodiments. Thus, the outer diameter designated OD of the first conductor 22 and first conductor sheath 23 is equal to SD plus TS plus TS, which is about 0.0072 inches, plus about 0.001 inches, plus about 0.001 inches, which equals about 0.0092 inches. In other preferred embodiments the outer diameter (OD) of the first conductor 22 and first conductor sheath 23 may be in the range from about 0.0102 inches to about 0.0082 inches.

As best shown in Figs. 3 and 4 (the latter figure being an enlarged view of outer strand 42), the outer wires 54, 56, 58, 60, 62 and 64, of each of the strands 40, 42, 44, 46, 48 and 50, and the core strand 52 are made of a first material 68. In one of the preferred embodiments the first material 68 comprises MP35N^{®}. MP35N^{®} is an alloy that includes nickel-cobalt-chromium-molybdenum and has a high tensile strength, good ductility, and corrosion resistance. MP35N^{®} is commercially available from SPS Technologies, Inc., Jenkintown, Pennsylvania. MP35N^{®} is well known to those having ordinary skill in the art and therefore it is not described herein in detail.

In another preferred embodiment the first material 68 may be 35N LT^{®}. 35N LT^{®} is an alloy that includes nickel-cobalt-chromium-molybdenum, with less titanium than MP35N^{®}. 35N LT^{®} is commercially available from Fort Wayne Metals Research Products Corp., Fort Wayne, Indiana.

The core wire 66 in each of the outer strands 40, 42, 44, 46, 48 and 50, and the core strand 52, has a core 67 centrally positioned in a tube 70, such that the core 67 and tube 70 are substantially concentric. The core 67 extends coaxially with the tube 70 and contacts the tube 70. In one of the preferred embodiments, the tube 70 is made of the previously described first material 68, and the core 67 is made of a second material 80. In another preferred embodiment, the tube 70 may be replaced with a layer of the first material 68 that surrounds the core 67. The second material 80 from which the core 67 is made is silver. In another preferred embodiment, the second material 80 from which the core 67 is made is a silver alloy, and the silver alloy may have a silver content of 28% by weight and a nickel-cobalt-chromium-molybdenum alloy content, or MP35N^{®} content, of about 72% by weight. In other preferred embodiments of the second material 80, the silver content by weight may be more or less than about 28% depending on the particular requirements of the application in which the cabled lead 20 will be employed.

It is to be understood that the core wires 66 in outer strands 40, 44, 46, 48 and 50, and core the strand 52 are substantially identical to the core wire 66 of the outer strand 42 described above. In addition, the core wires 66 may be made by drawn filled tube manufacturing techniques. Drawn filled tube and drawn filled tube manufacturing techniques are well known to those having ordinary skill in the art and are therefore not described.

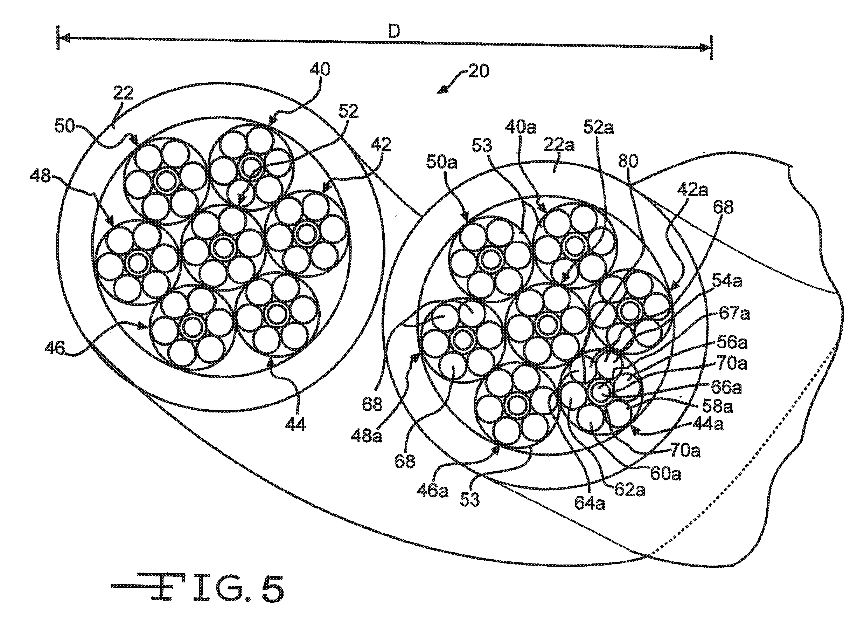

As best shown in Fig. 5, the second conductor cable 22a is substantially identical to the first conductor cable 22, and has six outer strands 40a, 42a, 44a, 46a, 48a and 50a, and a core strand 52a centrally positioned in the outer strands. The outer strands 40a, 42a, 44a, 46a, 48a and 50a may be helically wound around the core strand 52a. Each of the outer stands 40a, 42a, 44a, 46a, 48a and 50a, and the core strand 52a has six outer wires 54a, 56a, 58a, 60a, 62a and 64a that may be helically wound around a core wire 66a. In Fig. 5, outer strand 44a and outer wires 54a, 56a, 58a, 60a, 62a and 64a and core wire 66a are identified. It is to be understood that each of outer strands 40a, 42a, 46a and 50a, and core strand 52a has a substantially identical wire configuration as outer strand 44a, but have not been numbered for the sake of clarity.

Each of the outer strands 40a, 42a, 44a, 46a, 48a and 50a, and the core strand 52a may be surrounded by a strand insulation sheath 53. The outer wires 54a, 56a, 58a, 60a, 62a and 64a, in each of the strands 40a, 42a, 44a, 46a, 48a and 50a and core strand 52a are made of the previously described first material 68. The core wire 66a has a core 67a centrally positioned in a tube 70a, such that the core 67a and tube 70a are concentric. The core 67a extends coaxially with the tube 70a and contacts the tube 70a. In one of the preferred embodiments, the tube 70a is made of the previously described first material 68, and the core 67a is made of the previously described second material 80. In another preferred embodiment, the first material 68 may be in the form of a layer which surrounds the core 67a.

Thus, each of outer strands 40a, 42a, 44, 46a, 48a and 50a, and the core strand 52a has seven wires (six outer wires 52a, 54a, 56a, 58a, 60a and 62a, and one core wire 64a). The configuration of the second cable 22a may generally be described as being a 7 x 7 configuration, because there are six outer strands and the core strand (40a, 42a, 44a, 46a, 48a, 50a and 52a), each having six outer wires and a core wire (54a, 56a, 58a, 60a, 62a, 64a and 66a) for a combined total of 49 outer wires and core wires.

In one of the preferred embodiments, each of the outer wires 52a, 54a, 56a, 58a, 60a and 62a and the central wire 64a has the same dimensions as described in connection with the first conductor cable 22, the second conductor sheath 23a has the same thickness as first conductor sheath 23, and the overall diameter of the second conductor cable 22a is the same as the overall diameter of the first conductor cable 22.

The first and second conductor cables 22 and 22a are helically coiled such that the cabled lead 20 has the shape of a bifilar helical coil designated 25 in Fig. 2. The bifilar helical coil 25 extends in the direction of longitudinal axis X. In addition, the cabled lead 20 may be described as being blended, because each of the first and conductor cables 22 and 22a is made of the first and second materials 68 and 80, respectively. In one of the preferred embodiments, the cabled lead 20 has a length designated L of 20 inches. In other preferred embodiments, the length could be virtually any length depending on the application in which the cabled lead 20 will be employed. The pitch length, designated PL in Fig. 2, of the bifilar helical coil 25 is about 0.02 inches, and in other preferred embodiments may be about 0.02 inches. As used herein, the term pitch length means the axial distance for the first conductor cable 22 to make one 360 degree revolution about the longitudinal axis X.

As shown in Fig. 5, the diameter D of the cabled lead 20 is about 0.020 inches (diameter of the first conductor cable 22 and the first conductor sheath 23, plus the diameter of the second conductor cable 22a and the second conductor sheath 23a). In other preferred embodiments the diameter D of the cabled lead 20 is in the range from about 0.018 inches to about 0.022 inches.

The first and second conductor sheaths 23 and 23a surrounding the first and second conductor cables 22 and 22a may be any desired color to facilitate visual recognition. For example, the first conductor sheath 23 may be blue and the second conductor sheath 23a may be white, a natural color, or any desired color. This advantageously allows the physician to immediately recognize and visually distinguish the first and second conductor cables 22 and 22a.

The cabled lead 20 has many advantages including: a long working life, high flexibility, resistance to breaking, high strength, resistance to fatigue, and high conductivity. The cabled lead 20 may advantageously be made to any length as required for the body in which it will be implanted. For example, an elongated cabled lead 20 may be implanted in the spine. The cabled lead 20 may be subjected to constant movement of the body in which it is implanted and advantageously will not break due to fatigue or weaken over time. The strength and fatigue resistance of the cabled lead 20 is due in part to the great number of outer wires and core wires which total 98 (each of the first and second conductor cables has 49) from which the cabled lead 20 is made, and due in part to the first and second materials 68, 80 from which the cabled lead 20 is made. In addition, the diameter of the cabled lead 20 is advantageously small such that the surgical procedures associated with installation and removal of the cabled lead 20 may be characterized as having a very low level of invasiveness. And, because of its relatively small diameter, high resistance to fatigue, and its capacity to reliably deliver or detect electrical impulses, the cabled lead 20 is advantageously well suited for use in cardiac simulation, gastric stimulation, neurological stimulation and spinal cord stimulation applications.

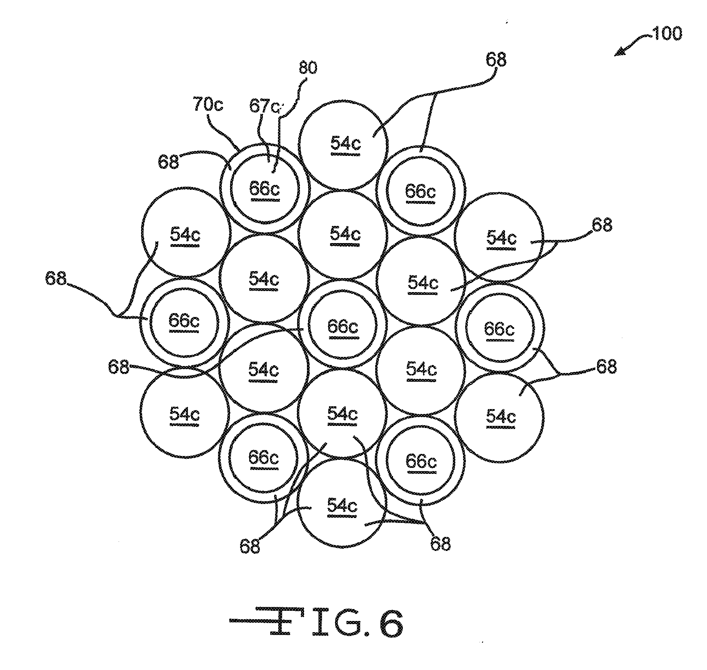

Fig. 6 is another preferred embodiment showing a multi-cored strand 100 having outer wires 54c which may be substantially structurally and dimensionally identical to outer wires 54 previously described, and core wires 66c which may be substantially structurally and dimensionally identical to the core wires 66 previously described. The outer wires 54c are made of the first material 68, and the core wires 66c have tubes 70c made of the first material 68 with cores 67c made of the second material 80. There are twelve outer wires 54c and seven core wires 66c, thus the multi-cored strand 100 may be described as having a 1 x 19 configuration. It is pointed out that the core wires 66c and the outer wires 54c are positioned such that the outer wires 54c contact outer wires 54c and core wires 66c, and the core wires 66c only contact outer wires 54c. This advantageously provides for equally distributed strength, conductivity, and flexibility throughout the multi-cored strand 100. A cabled lead may be provided wherein all the outer and core stands in the conductor cables 22 and 22a are replaced with multi-cored strands 100.

In another preferred embodiment there is only a single conductor cable 22 housed in the first conductor sheath 23, and the second conductor cable 22a is not present. The single conductor cable 22 may be helically coiled and used by itself for delivering electrical stimulation. In other preferred embodiments, there may be more than the first and second conductor cables 22 and 22a. For example, there may be a third conductor cable substantially structurally identical to the first and second conductor cables 22 and 22a, and each is housed in a conductor sheath 23. The three conductor cables housed in sheaths may be helically coiled to form a trifilar cabled lead. In another preferred embodiment, there may be a fourth conductor cable substantially identical to the first, second and third conductor cables, with each being housed in a sheath 23. The four conductor cables may be helically coiled to form a quadfilar cabled lead. Additional conductor cables housed in sheaths may be provided and helically coiled in the same manner as desired.

While the blended coiled cable 20 has been described in connection with certain embodiments, it is not intended to limit the scope of the invention to the particular forms set forth. On the contrary, the blended coiled cable invention is intended to cover such alternatives, modifications, and equivalents as may be included within the spirit and scope of the appended claims.

An aspect of the invention provides a method for making a conductor cable, comprising the steps of:
a) providing outer strands and providing a core strand and centrally positioning the core strand in the outer strands;
b) providing each of the outer strands and the core strand with outer wires and a core wire and positioning the outer wires around a core wire;
c) providing a first material and forming the outer wires from the first material;
d) providing a second material; and
e) forming the core wire from a tube made of the first material and positioning a core made of the second material in the tube.

Said method may include providing the second material to include silver.

A further aspect of the invention provides a method for making a cabled lead, comprising the steps of:
a) providing a first conductor cable;
b) providing a second conductor cable;
c) providing each of the first and second conductor cables with outer strands and a core strand and positioning the core strand such that it is surrounded by the outer strands;
d) providing each of the outer strands and core strand with outer wires and a centrally positioned core wire;
e) providing a first material and forming the outer wires from the first material;
f) providing a second material; and
g) forming each of the core wires of each of the outer strands and core strands from a tube made of the first material, and providing the tube with a core made of the second material.

Said method may include providing the number of outer strands in each of the first and second conductor cables to be six, and the number of core strands in each of the first and second conductor cables to be one.

Said method may include positioning the first conductor cable in a first conductor sheath and positioning the second conductor cable in a second conductor sheath.

Said method may include providing strand insulation sheaths and positioning each of the outer strands and core strands in one of the strand insulation sheaths.

In said method the first material may be a nickel-cobalt-chromium-molybdenum alloy.

Said method may include providing the second material to include silver.

Said method may include providing the first material to be about 78% by weight an alloy of nickel-cobalt-chromium-molybdenum alloy and providing the second material to be about 28% by weight silver.

## Claims

1. A conductor cable comprising:
a) outer strands; and
b) a core strand centrally positioned in the outer strands such that the core strand is surrounded by the outer strands;
c) each of the outer strands and the core strand having outer wires that surround a core wire, and the outer wires being made of a first material; and
d) each of the core wires having a tube made of the first material and a core made of a second material, and the core being positioned in the tube.

2. The conductor cable of claim 1 wherein the outer wires are made of only the first material.

3. The conductor cable of claim 1 or claim 2 wherein the first material includes a nickel-cobalt-chromium-molybdenum alloy.

4. The conductor cable of any of claims 1 to 3 wherein the second material includes silver.

5. The conductor cable of any of claims 1 to 4 wherein the first material includes a nickel-cobalt-chromium-molybdenum alloy and the second material is a silver alloy.

6. The conductor cable of any of claims 1 to 5 wherein each of the cores have a silver content of about 28% by weight and a nickel-cobalt-chromium-molybdenum alloy content of about 72% by weight.

7. The conductor cable of any of claims 1 to 6 wherein the diameter of each of the outer wires and core wire is about 0.0008 inches.

8. The conductor cable of any of claims 1 to 7 further including strand insulation sheaths wherein each of the outer strands and the core strand is positioned in one of the strand insulation sheaths.

9. The conductor cable of any of claims 1 to 8 further including a conductor sheath wherein the conductor cable is positioned in the conductor sheath.

10. The conductor cable of claim 9 wherein the conductor sheath has a thickness of about 0.001 inches.

11. A cabled lead, which comprises:
a) a first conductor cable; and
b) a second conductor cable;
wherein the first and second conductor cables are as defined in any of claims 1 to 10.

12. The cabled lead of claim 11, which comprises:
a) a first conductor cable and a second conductor cable, each having six outer strands and one core strand centrally positioned in each of the six outer strands;
b) wherein each of the six outer strands and the core strand has six outer wires and a core wire such that the core wire is centrally positioned in the six outer wires;
c) wherein the six outer wires of each of the six outer strands and the core strand are made of the first material; and
d) wherein each of the core wires comprises a tube made of the first material and a core made of the second material, and wherein the core is positioned in the tube.

13. The cabled lead of claim 12 wherein the first conductor cable is positioned in a first conductor sheath and the second conductor cable is positioned in a second conductor sheath and wherein the combined diameter of the first conductor cable and the first conductor sheath and the second conductor cable and the second conductor sheath is in the range from about 0.018 inches to about 0.022 inches.

14. The cabled lead of claim 12 wherein the first and second conductor cables are helically coiled to form a bifilar coil.

15. A conductor cable, which comprises:
a) a strand having outer wires and core wires;
b) wherein the outer wires are made of a first material;
c) the core wires having tubes made of the first material with cores made of a second material positioned in the tubes; and
d) wherein the outer wires contact outer wires and core wires, and the core wires only contact outer wires.

16. A method for making a conductor cable, comprising the steps of:
a) providing outer strands and providing a core strand and centrally positioning the core strand in the outer strands;
b) providing each of the outer strands and the core strand with outer wires and a core wire and positioning the outer wires around a core wire;
c) providing a first material and forming the outer wires from the first material;
d) providing a second material; and
e) forming the core wire from a tube made of the first material and positioning a core made of the second material in the tube.

17. A method for making a cabled lead, comprising the steps of:
a) providing a first conductor cable;
b) providing a second conductor cable;
c) providing each of the first and second conductor cables with outer strands and a core strand and positioning the core strand such that it is surrounded by the outer strands;
d) providing each of the outer strands and core strand with outer wires and a centrally positioned core wire;
e) providing a first material and forming the outer wires from the first material;
f) providing a second material; and
g) forming each of the core wires of each of the outer strands and core strands from a tube made of the first material, and providing the tube with a core made of the second material.
